# EUROPEAN PATENT APPLICATION

(11) **EP 3 329 874 A1**
(43) Date of publication of application: **06.06.2018**
(21) Application number: 16900399.3
(22) Date of filing: 26.04.2016
(51) Int. Cl.: A61B 18/14

(54) **ENERGY TREATMENT TOOL, TREATMENT SYSTEM, AND CONTROL DEVICE**

(71) Applicant: Olympus Corporation, Hachioji-shi, Tokyo 192-8507 (JP)
(72) Inventor: HAYASHIDA, Tsuyoshi, Hachioji-shi Tokyo 192-8507 (JP); SAKAO, Satomi, Hachioji-shi Tokyo 192-8507 (JP)
(74) Representative: Gunzelmann, Rainer
(86) International application number: PCT/JP2016/063093
(87) International publication number: WO 2017/187526

(57) **Abstract**

An energy treatment instrument includes a first grasping piece, and a second grasping piece which opens or closes relative to the first grasping piece and which grasps a blood vessel between the first grasping piece and the second grasping piece. An actuation state of the energy treatment instrument is switched between a first mode to coagulate the blood vessel when a branch of the blood vessel is not present within a predetermined range from a position where the blood vessel is grasped, and a second mode to coagulate the blood vessel when the branch of the blood vessel is present within the predetermined range.

## Description

### Technical Field

The present invention relates to an energy treatment instrument which applies treatment energy to a treated target grasped between a pair of grasping pieces, a treatment system comprising the energy treatment instrument, and a controller which is used together with the energy treatment instrument.

### Background Art

International Publication No. 2012/061638 discloses an energy treatment instrument which grasps a treated target such as a living tissue between a pair of grasping pieces. In this energy treatment instrument, an electrode is provided in each of the grasping pieces. By the supply of electric energy to both of the electrodes, a high-frequency current flows between the electrodes through the grasped treated target. Thereby, the high-frequency current is applied to the treated target as treatment energy.

### Summary of Invention

When a treatment is conducted by use of an energy treatment instrument such as the one in International Publication No. 2012/061638, a branched portion (a branch or the vicinity of the branch) of a blood vessel may be grasped as a treated target between a pair of grasping pieces, and the blood vessel may be sealed. In such a treatment, if the treatment is conducted as in the case where the blood vessel is grasped in a portion other than the branched portion, there is concern that a treatment to seal the blood vessel by use of treatment energy may be affected. Accordingly, there is a possibility that performance of sealing the blood vessel, such as a pressure resistance value (difficulty of the flow of blood) of the sealed blood vessel, may be affected.

The present invention has been made to solve the problems described above, and its object is to provide a treatment system and a controller wherein suitable treatment performance can be achieved even if a blood vessel is grasped in a branched portion.

To solve the above mentioned problems, according to one aspect of the invention, An energy treatment instrument including a first grasping piece, and a second grasping piece which opens or closes relative to the first grasping piece and which grasps a blood vessel between the first grasping piece and the second grasping piece, wherein an actuation state of the energy treatment instrument is switched between a first mode to coagulate the blood vessel when a branch of the blood vessel is not present within a predetermined range from a position where the blood vessel is grasped, and a second mode to coagulate the blood vessel when the branch of the blood vessel is present within the predetermined range.

According to one another aspect of the invention, a controller which is used together with an energy treatment instrument, the energy treatment instrument including a first grasping piece, and a second grasping piece which opens or closes relative to the first grasping piece and which grasps a blood vessel between the first grasping piece and the second grasping piece, the controller including, an energy output source which outputs electric energy that is supplied to the energy treatment instrument, and which applies treatment energy to the blood vessel grasped between the first grasping piece and the second grasping piece by the supply of the electric energy to the energy treatment instrument, and a processor which judges, on the basis of an observation image resulting from observation by an observation element, whether or not any branch of the blood vessel is present within the predetermined range from the position where the blood vessel is grasped, the processor performing at least one of the following, controlling the output of the electric energy from the energy output source on the basis of a judgement result of the branch, and making force of grasping the blood vessel between the first grasping piece and the second grasping piece greater when it is judged that the branch is present than when it is judged that the branch is not present.

### Brief Description of Drawings

FIG. 1 is a schematic diagram showing a treatment system according to a first embodiment;
FIG. 2 is a block diagram showing a control configuration in the treatment system according to the first embodiment;
FIG. 3 is a flowchart showing processing at processors in a sealing treatment of a blood vessel using the treatment system according to the first embodiment;
FIG. 4 is a flowchart showing processing in output control in a first sealing mode by the processor according to the first embodiment;
FIG. 5 is a schematic diagram showing one example of a change with time of impedance between a pair of grasping pieces in a state where the processor according to the first embodiment is performing output control in each of the first and second sealing modes;
FIG. 6 is a schematic diagram showing one example of an observation image in a state where the a branched portion (the vicinity of the branch) of the blood vessel is grasped between the grasping pieces according to the first embodiment;
FIG. 7 is a schematic diagram showing one example of a change with time of the impedance between the pair of grasping pieces in a state where the processor according to a first modification of the first embodiment is performing output control in each of the first and second sealing modes;
FIG. 8 is a flowchart showing processing in output control in the second sealing mode by the processor according to a second modification of the first embodiment;
FIG. 9 is a schematic diagram showing one example of a change with time of the impedance between the pair of grasping pieces in a state where the processor according to the second modification of the first embodiment is performing output control in each of the first and second sealing modes;
FIG. 10 is a flowchart showing processing in output control in the second sealing mode by the processor according to a third modification of the first embodiment;
FIG. 11 is a flowchart showing processing at the processors in the sealing treatment of the blood vessel using the treatment system according to a fourth modification of the first embodiment;
FIG. 12 is a block diagram showing a control configuration in a treatment system according to a second embodiment;
FIG. 13 is a schematic diagram showing one example of a grasping force adjustment element according to the second embodiment;
FIG. 14 is a flowchart showing processing at the processors in a sealing treatment of the blood vessel using the treatment system according to the second embodiment; and
FIG. 15 is a flowchart showing processing at the processor in a sealing treatment of the blood vessel using a treatment system according to a certain modification of the first and second embodiments.

### Description of Embodiments

### (First Embodiment)

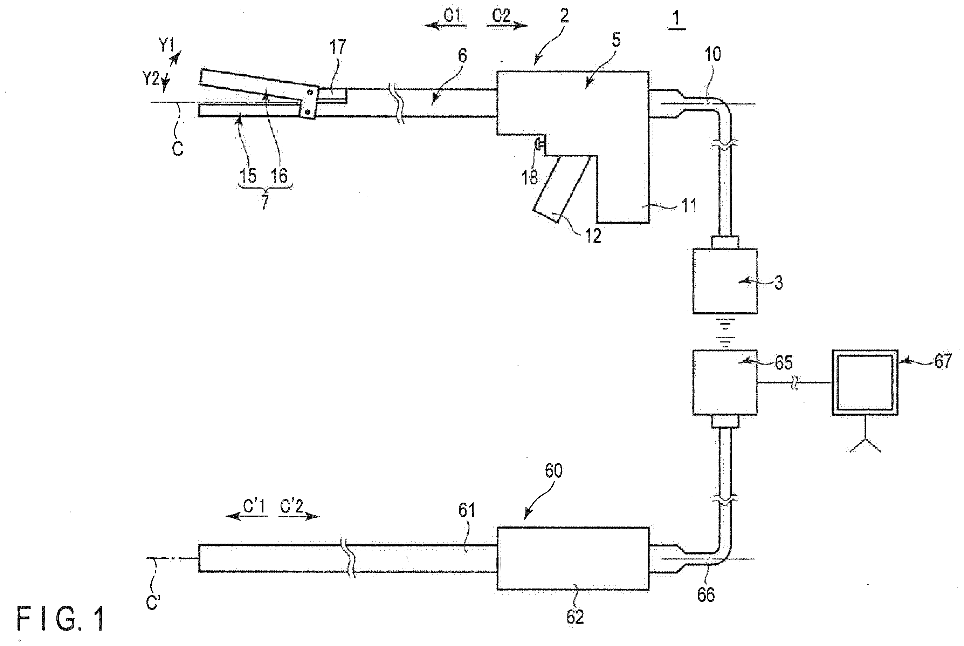

A first embodiment of the present invention is described with reference to FIG. 1 to FIG. 6. FIG. 1 is a diagram showing a treatment system 1 according to the present embodiment. As shown in FIG. 1, the treatment system 1 comprises an energy treatment instrument 2 and a controller (energy controller) 3. The energy treatment instrument 2 has a longitudinal axis C. Here, in the energy treatment instrument 2, one side in a direction along the longitudinal axis C is a distal side (an arrow C1 side), and the side opposite to the distal side is a proximal side (an arrow C2 side).

The energy treatment instrument 2 comprises a housing 5 which is holdable, a sheath (shaft) 6 which is coupled to the distal side of the housing 5, and an end effector 7 provided in a distal portion of the sheath 6. One end of a cable 10 is connected to the housing 5 of the energy treatment instrument 2. The other end of the cable 10 is separably connected to the controller 3. Further, a grip (fixed handle) 11 is provided in the housing 5, and a handle (movable handle) 12 is revolvably attached to the housing 5. The handle 12 revolves relative to the housing 5, and the handle 12 opens or closes relative to the grip 11. Note that in the present embodiment, the handle 12 is located on the distal side of the grip 11, and moves substantially parallel to the longitudinal axis C in an operation of opening or closing relative to the grip 11, which is, however, not restrictive. For example, in a certain example, the handle 12 may be located on the proximal side of the grip 11. In another certain example, the handle 12 may be located on the side opposite to the grip 11 across the longitudinal axis C, and the movement direction of the handle 12 in the operation of opening or closing relative to the grip 11 may cross (may be substantially perpendicular to) the longitudinal axis C.

The sheath 6 extends along the longitudinal axis C. Further, the end effector 7 comprises a first grasping piece 15, and a second grasping piece 16 which opens or closes relative to the first grasping piece 15. The handle 12 and the end effector 7 are coupled to each other via a movable member 17 extending along the longitudinal axis C through the sheath 6. The handle 12 which is an open/close operation input section is opened or closed relative to the grip 11, whereby the movable member 17 moves relative to the sheath 6 and the housing 5 along the longitudinal axis C, and the pair of grasping pieces 15 and 16 open or close relative to each other. The grasping pieces 15 and 16 close relative to each other, and a living tissue such as a blood vessel is thereby grasped as a treated target between the grasping pieces 15 and 16. Open/close directions (directions of an arrow Y1 and an arrow Y2) of each of the grasping pieces 15 and 16 cross (are substantially perpendicular to) the longitudinal axis C.

Note that the end effector 7 has only to be configured so that the pair of grasping pieces 15 and 16 open or close relative to each other in response to each of the open and close operations of the handle 12. For example, in a certain example, one of the grasping pieces 15 and 16 is integral with the sheath 6 or fixed to the sheath 6, and the other of the grasping pieces 15 and 16 is revolvably attached to the distal portion of the sheath 6. In another certain example, both of the grasping pieces 15 and 16 are revolvably attached to the distal portion of the sheath 6. In yet another certain example, a rod member (not shown) is inserted through the sheath 6, and one of the grasping pieces 15 and 16 is formed by a portion of the rod member (probe) protruding toward the distal side from the sheath 6. Further, the other of the grasping pieces 15 and 16 is revolvably attached to the distal portion of the sheath 6. Moreover, in a certain example, a rotational operation knob (not shown) may be attached to the housing 5. In this case, the rotational operation knob is rotated around the longitudinal axis C relative to the housing 5, whereby the sheath 6 and the end effector 7 rotate around the longitudinal axis C relative to the housing 5 together with the rotational operation knob. Accordingly, the angular position of the end effector 7 around the longitudinal axis C is adjusted.

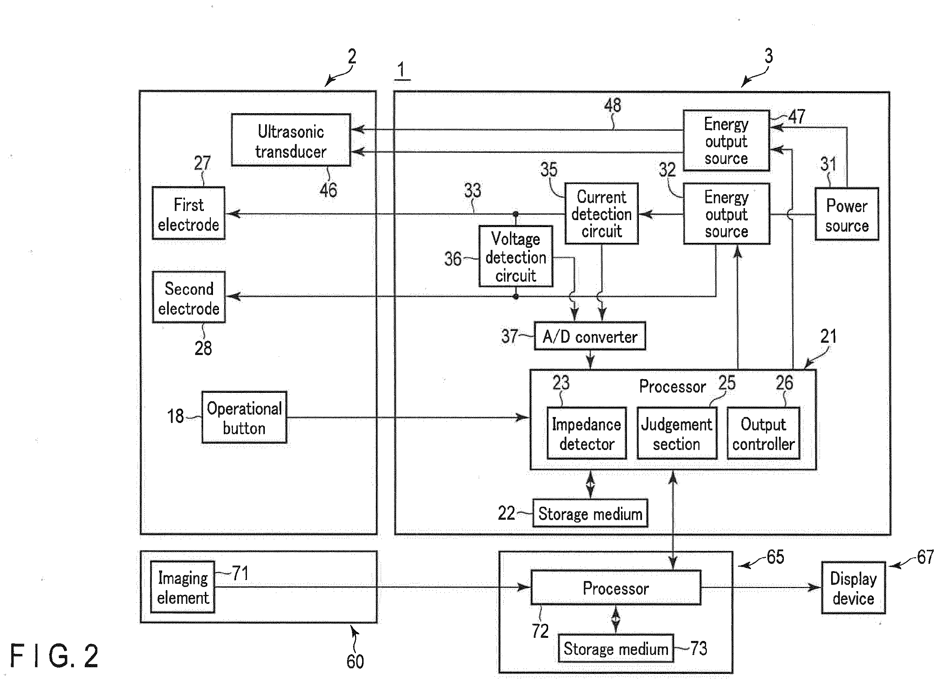

FIG. 2 is a diagram showing a control configuration in the treatment system 1. As shown in FIG. 2, the controller 3 comprises a processor (control section) 21 which controls the whole treatment system 1, and a storage medium 22. The processor 21 is formed from an integrated circuit including a central processing unit (CPU), an application specific integrated circuit (ASIC) or a field programmable gate array (FPGA), and the like. The processor 21 may be formed from one integrated circuit or may be formed from more than one integrated circuit. The processing in the processor 21 is performed in accordance with a program stored in the processor 21 or the storage medium 22. Further, a processing program for use in the processor 21, parameters and a table for use in the calculation in the processor 21, and others are stored in the storage medium 22. The processor 21 comprises an impedance detector 23, a judgement section 25, and an output controller 26. The impedance detector 23, the judgement section 25, and the output controller 26 function as parts of the processor 21, and perform parts of the processing performed by the processor 21.

In the end effector 7 of the energy treatment instrument 2, a first electrode 27 is provided in the first grasping piece 15, and a second electrode 28 is provided in the second grasping piece 16. The electrodes 27 and 28 are made of an electrically conductive material. The controller 3 comprises a power source 31 which is a battery, an outlet, or the like, and an energy output source (first energy output source) 32. The energy output source 32 is electrically connected to the electrodes 27 and 28 via an electricity supply path (first electricity supply path) 33 extending through the cable 10. The energy output source 32 comprises a conversion circuit, an amplifier circuit, and others, and converts electric power from the power source 31. Then the energy output source 32 outputs electric energy (high-frequency electric power) resulting from the conversion. The electric energy output from the energy output source 32 is supplied to the electrodes 27 and 28 through the electricity supply path 33. The output controller 26 of the processor 21 controls driving of the energy output source 32, and controls the output of the electric energy from the energy output source 32. Thereby, one of output electric power P, an output current I, and an output voltage V in the energy output source 32 is adjusted, and the supply of the electric energy to the electrodes 27 and 28 is controlled.

The electric energy is supplied to the electrodes 27 and 28 from the energy output source 32 in a state where the treated target is grasped between the grasping pieces 15 and 16, whereby a high-frequency current flows between the electrodes 27 and 28 through the treated target grasped in contact with the electrodes 27 and 28. That is, the high-frequency current is applied to the treated target as treatment energy. The high-frequency current flows through the treated target, whereby heat is generated in the treated target, and the treated target is denatured by the heat. Accordingly, the treated target which is a blood vessel or the like is sealed (coagulated) by use of the high-frequency current. As described above, by the supply of the electric energy to the electrodes 27 and 28 of the energy treatment instrument 2 from the energy output source 32, the treatment energy (high-frequency current) is applied to the treated target grasped between the grasping pieces 15 and 16. Therefore, in the present embodiment, the grasping pieces 15 and 16 are an energy application section which applies the high-frequency current to the grasped treated target (blood vessel) as the treatment energy.

A current detection circuit 35 and a voltage detection circuit 36 are provided in the electricity supply path 33. In a state where the electric energy is output from the energy output source 32, the current detection circuit 35 detects the output current I, and the voltage detection circuit 36 detects the output voltage V. An A/D converter 37 is provided in the energy controller 3. An analog signal regarding the current I detected in the current detection circuit 35, and an analog signal regarding the voltage V detected in the voltage detection circuit 36 are transmitted to the A/D converter 37. The A/D converter 37 converts the analog signal regarding the current I and the analog signal regarding the voltage V into digital signals, and transmits the digital signals resulting from the conversion to the processor 21.

In a state where the electric energy is output from the energy output source 32, the processor 21 acquires information regarding the output current I and the output voltage V in the energy output source 32. Further, the impedance detector 23 of the processor 21 detects impedance of the electricity supply path 33 including the grasped treated target (blood vessel) and the electrodes 27 and 28 on the basis of the output current I and the output voltage V. Thereby, impedance Z between the pair of grasping pieces 15 and 16 (i.e., impedance of the grasped treated target) is detected.

As shown in FIG. 1, an operational button 18 is attached to the housing 5 as an energy operation input section. By the pressing of the operational button 18, an operation (signal) to output the electric energy to the energy treatment instrument 2 from the energy output source 32 is input to the controller 3. Note that a foot switch or the like separate from the energy treatment instrument 2 may be provided as the energy operation input section instead of or in addition to the operational button 18. As shown in FIG. 2, the processor 21 detects whether or not there is any operation input in the energy operation input section such as the operational button 18. The output controller 26 of the processor 21 controls the output of the electric energy from the energy output source 32 on the basis of the operation input with the operational button 18.

As shown in FIG. 1 and FIG. 2, the treatment system 1 comprises a rigid endoscope (endoscope) 60 as an observation element (observation instrument). The rigid endoscope 60 has a longitudinal axis C'. Here, in the rigid endoscope 60, one side in a direction along the longitudinal axis C' is a distal side (an arrow C1' side), and the side opposite to the distal side is a proximal side (an arrow C2' side). The rigid endoscope 60 comprises an insertion section 61 extending along the longitudinal axis C', and a holdable holding portion 62 provided on the proximal side of the insertion section 61. Further, the treatment system 1 comprises an image processing device 65 and a display device 67 such as a monitor. One end of a universal cord 66 is connected to the holding portion 62 of the rigid endoscope 60. The other end of the universal cord 66 is separably connected to the image processing device 65. The image processing device 65 is electrically connected to the display device 67.

An imaging element 71 such as a CCD is provided in a distal portion of the insertion section 61 of the rigid endoscope 60. A subject is imaged by the imaging element 71. For example, in a state where the blood vessel (treated target) is grasped between the grasping pieces 15 and 16, the grasped blood vessel, the grasping pieces 15 and 16 (the end effector 7), and others are imaged as the subject by the imaging element 71. In this instance, the imaging element 71 performs, for example, stereoscopic photography. Moreover, the imaging of the subject by the imaging element 71 is continuously performed with time. As described above, the grasped blood vessel is observed by use of the rigid endoscope 60.

The image processing device 65 comprises a processor (image processing unit) 72 which performs image processing and the like, and a storage medium 73. The processor 72 is formed from an integrated circuit including a CPU, an ASIC or an FPGA, and the like. The processor 72 may be formed from one integrated circuit or may be formed from more than one integrated circuit. The processing in the processor 72 is performed in accordance with a program stored in the processor 72 or the storage medium 73. Further, a processing program for use in the processor 72, parameters and a table for use in the calculation in the processor 72, and others are stored in the storage medium 73. The processor 72 is capable of communicating (capable of exchanging information) with the processor 21 of the controller 3 in a wired or wireless way.

When the subject is imaged by the imaging element 71, an image signal is transmitted to the processor 72. Thereby, the processor 72 generates an observation image of a subject such as the grasped blood vessel. In this instance, if the stereoscopic photography is performed in the imaging element 71, the processor 72 generates a three-dimensional image as the observation image of the subject. In addition, because the subject is continuously imaged with time, observation images are continuously generated with time in the processor 72. The observation image generated in the processor 72 is displayed on the display device 67.

Furthermore, the processor 72 performs image processing and thereby acquires information regarding the subject from the generated observation image. For example, the processor 72 identifies the positions of the grasping pieces 15 and 16 and the blood vessel in the observation image on the basis of the luminance, colors, or the like of pixels that form the observation image. Further, in the observation image, the position where the blood vessel is grasped by the grasping pieces 15 and 16 (the grasping position of the blood vessel) is identified. Then the processor 72 detects, in the observation image, a branch of the blood vessel using, as a detection range, a predetermined range around the position where the blood vessel is grasped (the grasping position of the blood vessel). Here, the predetermined range is, for example, a range which is at a predetermined distance Lth or less from the grasping position of the blood vessel, in which case the predetermined distance Lth is stored in the storage medium 73 or the like. In the processing to detect the branch of the blood vessel, thinning processing of the blood vessel is performed on the basis of an obtained blood vessel image (position information), for example, as shown in Japanese Patent No. 2011-167529. By the thinning processing, a blood vessel portion is segmentalized in the observation image. Further, the processor 72 detects a branch point in the segments generated by the thinning processing. Then the processor 72 detects (extracts) the branch point in the segments as the branch of the blood vessel. Image data for the observation image generated in the processor 72 are transmitted to the processor 21 of the controller 3, and the position where the blood vessel is grasped (the grasping position of the blood vessel) in the observation image and results of the image processing in the processor 72 such as a detection result of the branch point are also transmitted to the processor 21 of the controller 3.

The judgement section 25 of the processor 21 judges whether or not any branch of the blood vessel is present within the predetermined range from the position where the blood vessel is grasped (the grasping position of the blood vessel), on the basis of the generated image data for the observation image and the result of the image processing in the processor 72. That is, the judgement section 25 judges whether or not the portion grasped between the grasping pieces 15 and 16 is the branched portion (the branch or the vicinity of the branch) of the blood vessel. Then the output controller 26 of the processor 21 controls the output of the electric energy from the energy output source 32 on the basis of the judgement result regarding the branch. The actuation state of the energy treatment instrument 2 switches between a first mode (first actuation mode) and a second mode (second actuation mode) in response to the output state of the electric energy from the energy output source 32. In the present embodiment, the state of the application of the treatment energy (high-frequency current) to the grasped treated target (blood vessel) from the energy application section (the grasping pieces 15 and 16) varies between the first mode and the second mode.

Note that in a certain example, an ultrasonic transducer 46 may be provided in the energy treatment instrument 2 (inside the housing 5). In this case, the rod member is connected to the distal side of the ultrasonic transducer 46, and one of the grasping pieces 15 and 16 (e.g., the first grasping piece 15) is formed by a portion of the rod member protruding toward the distal side from the sheath 6. Moreover, in the present example, an energy output source (second energy output source) 47 is provided in the controller 3 in addition to the energy output source 32. The energy output source 47 is electrically connected to the ultrasonic transducer 46 via an electricity supply path (second electricity supply path) 48 extending through the cable 10. Here, the energy output source 47 may be integral with the energy output source 32, or may be formed separately from the energy output source 32.

In the present example, the energy output source 47 comprises a conversion circuit, an amplifier circuit, and others, and converts electric power from the power source 31. Then the energy output source 47 outputs electric energy (alternating-current electric power) resulting from the conversion. The electric energy output from the energy output source 47 is supplied to the ultrasonic transducer 46 through the electricity supply path 48. The output controller 26 of the processor 21 controls driving of the energy output source 47, and controls the output of the electric energy from the energy output source 47.

In the present example, the electric energy (alternating-current electric power) output from the energy output source 47 is supplied to the ultrasonic transducer 46, and ultrasonic vibration is thereby generated in the ultrasonic transducer 46. The generated ultrasonic vibration is transmitted to the distal side from the proximal side in the rod member (vibration transmitting member), and the rod member including one of the grasping pieces 15 and 16 (e.g., the first grasping piece 15) vibrates. The rod member vibrates in a state where the treated target is grasped between the grasping pieces 15 and 16, whereby the ultrasonic vibration is applied to the treated target as the treatment energy. In this instance, frictional heat resulting from the vibration is generated, and the treated target which is the blood vessel or the like can be cut open while being sealed (coagulated) by the frictional heat.

In another certain example, a heater (not shown) may be provided in the end effector 7 (at least one of the grasping pieces 15 and 16) instead of the ultrasonic transducer 46. In this case, the electric energy (direct-current electric power or alternating-current electric power) output from the energy output source (47) is supplied to the heater through the electricity supply path (48). Thereby, heat is generated in the heater, and the treated target which is the blood vessel or the like can be cut open while being sealed (coagulated) by the heat generated in the heater. Even when each of the ultrasonic vibration and the heater heat or the like is applied to the grasped treated target (blood vessel) as the treatment energy, at least one of the grasping pieces 15 and 16 functions as the energy application section which applies the treatment energy to the treated target.

Now, functions and advantageous effects according to the present embodiment are described. When conducting a treatment using the treatment system 1, a surgeon holds the housing 5 of the energy treatment instrument 2, and inserts the end effector 7 into a body cavity such as an abdominal cavity. Further, a blood vessel (treated target) is disposed between the grasping pieces 15 and 16, the handle 12 is closed relative to the grip 11, and the grasping pieces 15 and 16 are thereby closed relative to each other. Accordingly, the blood vessel is grasped between the grasping pieces 15 and 16. In this instance, the insertion section 61 of the rigid endoscope 60 is also inserted into the body cavity, and the imaging element 71 continuously images, with time, the grasped blood vessel and the grasping pieces 15 and 16 as a subject. Thereby, the grasped blood vessel is observed. Further, for example, a high-frequency current is applied to the blood vessel as the treatment energy, and a sealing treatment of the grasped blood vessel is conducted.

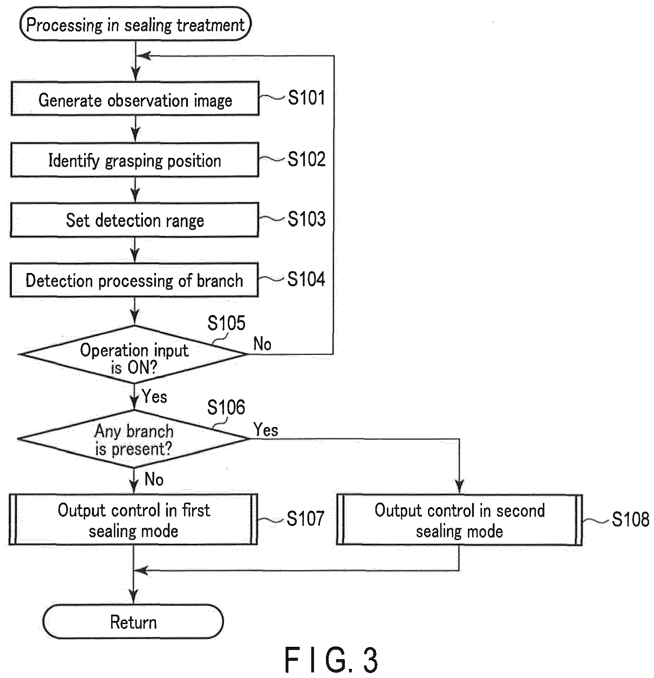

FIG. 3 is a flowchart showing processing at the processors 21 and 72 in a sealing treatment of the blood vessel using the treatment system 1 according to the present embodiment. As shown in FIG. 3, when conducting the sealing treatment of the blood vessel, the processor 72 generates an observation image on the basis of a subject image obtained by the imaging element 71 (step S101). Then the processor 72 identifies the positions of the grasping pieces 15 and 16 and the grasped blood vessel in the observation image on the basis of the luminance, colors, or the like of pixels that form the observation image. Note that a marker or the like may be attached to the end effector 7, and the positions of the grasping pieces 15 and 16 in the observation image may be identified on the basis of the position of the marker. Then the processor 72 identifies the position where the blood vessel is grasped (the grasping position of the blood vessel) in the observation image (step S102). In this instance, a display screen of the display device 67 may be a touch panel, and an operation indicating the position where the blood vessel is grasped in the observation image may be input by the surgeon or the like using the touch panel of the display device 67. In this case, the processor 72 identifies the position where the blood vessel is grasped (the grasping position of the blood vessel) in the observation image on the basis of an operation input in the touch panel. Further, the processor 72 sets a detection range to detect a branch of the blood vessel in the observation image, as a predetermined range around the position where the blood vessel is grasped (the grasping position of the blood vessel) (step S103). In this instance, for example, a range which is at the predetermined distance Lth or less from the grasping position of the blood vessel is set as the detection range. Then the processor 72 performs detection processing of the branch of the blood vessel in the set detection range (step S104). Note that the detection processing of the branch is performed as in, for example, Japanese Patent No. 2011-167529, as described above.

Furthermore, the processor 21 of the controller 3 judges whether or not an operation input with the operational button (energy operation input section) 18 is performed (i.e., whether an operation input is on or off) (step S105). When the operation input is not performed (step S105 - No), the processing returns to step S101, and the processing in and after step S101 is sequentially performed. Thus, the generation of the observation image and the detection processing of the branch of the blood vessel in the set detection range are repeated. When the operation input is performed (step S105 - Yes), the judgement section 25 of the processor 21 judges whether or not any branch of the blood vessel is present within the predetermined range from the position where the blood vessel is grasped (the grasping position of the blood vessel), on the basis of the detection result in the detection processing of the branch of the blood vessel (step S106). In this instance, the judgement is made on the basis of the observation image and the detection result in the detection processing of the branch of the blood vessel at the point where the operation input is switched on from an off-state or at a point nearest the former point.

When it is judged that the branch of the blood vessel is not present within the predetermined range (step S106 - No), the output controller 26 of the processor 21 performs output control of the electric energy from the energy output source 32 in a first sealing mode (step S107). When it is judged that a branch of the blood vessel is present within the predetermined range from the position where the blood vessel is grasped (step S106 - Yes), the output controller 26 performs output control of the electric energy from the energy output source 32 in a second sealing mode different from the first sealing mode (step S108). Note that in a state where the operation input with the operational button (energy operation input section) 18 is performed and the treatment energy is applied to the grasped blood vessel as well, the processor 72 generates an observation image on the basis of a subject image obtained by the imaging element 71.

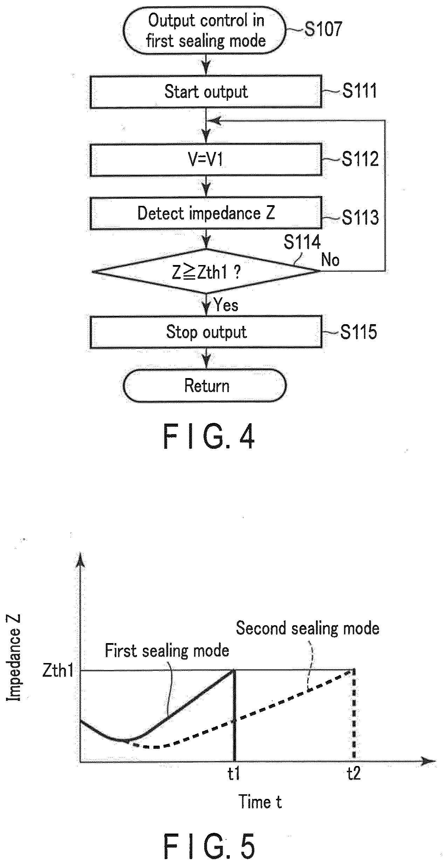

FIG. 4 is a flowchart showing processing by the processor 21 in the output control in the first sealing mode. As shown in FIG. 4, in the output control in the first sealing mode, the processor 21 starts the output of the electric energy (high-frequency electric power) from the energy output source (first energy output source) 32 (step Sill). Accordingly, the electric energy is supplied to the electrodes 27 and 28, a high-frequency current flows to the grasped blood vessel, and the blood vessel is sealed.

When a given length of time elapses from the start of the output of the electric energy from the energy output source 32, the output controller 26 performs constant voltage control to maintain, with time, the output voltage V from the energy output source 32 at a constant level of a first voltage value V1 (step S112). Moreover, when the output of the electric energy from the energy output source 32 is started, the impedance detector 23 of the processor 21 detects the impedance Z between the grasping pieces 15 and 16 (i.e., impedance of the grasped treated target) on the basis of the detection result of the output current I in the current detection circuit 35 and the detection result of the output voltage V in the voltage detection circuit 36 (step S113). Then the processor 21 judges whether or not the detected impedance Z is equal to or more than an impedance threshold (first impedance threshold) Zth1 (step S114). The impedance threshold Zth1 may be set by the surgeon or the like, or may be stored in the storage medium 22.

When the impedance Z is lower than the impedance threshold Zth1 (step S114 - No), the processing returns to step S112, and the processing in and after step S112 is sequentially performed. When the impedance Z is equal to or more than the impedance threshold Zth1 (step S114 - Yes), the output controller 26 stops the output of the electric energy (high-frequency electric power) from the energy output source 32 (step S115). Thereby, the supply of the electric energy to the electrodes 27 and 28 is stopped. The processor 21 performs the output control of the electric energy from the energy output source 32 in the first sealing mode, and the energy treatment instrument 2 is thereby actuated in the first mode to coagulate the grasped blood vessel. In the case where the blood vessel is coagulated when the branch of the blood vessel is not present within the predetermined range from the position where the blood vessel is grasped, the energy treatment instrument 2 is actuated in the first mode.

In the output control in the second sealing mode as well as in the output control in the first sealing mode, the processor 21 performs the processing in steps Sill and S113 to S115. However, in the second sealing mode, when a given length of time elapses from the start of the output of the electric energy from the energy output source 32, the output controller 26 performs constant voltage control to maintain, with time, the output voltage V from the energy output source 32 at a constant level of a second voltage value V2 lower than the first voltage value V1. Because the constant voltage control is performed at the second voltage value V2 lower than the first voltage value VI, the electric energy output from the energy output source 32 is lower in the second sealing mode than in the first sealing mode. That is, the output controller 26 of the processor 21 makes the electric energy output from the energy output source 32 lower in the second sealing mode than in the first sealing mode. The processor 21 performs the output control of the electric energy from the energy output source 32 in the second sealing mode, and the energy treatment instrument 2 thereby coagulates the grasped blood vessel and is actuated in the second mode different from the first mode. In the case where the blood vessel is coagulated when a branch of the blood vessel is present within the predetermined range from the position where the blood vessel is grasped, the energy treatment instrument 2 is actuated in the second mode. As described above, in the present embodiment, the processor 21 switches the actuation state of the energy treatment instrument 2 between the first mode (first actuation mode) and the second mode (second actuation mode) by controlling the output of the electric energy from the energy output source 32 on the basis of the judgement result of the branch of the blood vessel. The output state of the electric energy from the energy output source 32 varies between the first sealing mode and the second sealing mode, so that in the energy treatment instrument 2, the state of the application of the treatment energy (high-frequency current) to the grasped treated target (blood vessel) from the energy application section (the grasping pieces 15 and 16) varies between the first mode and the second mode.

Note that if the electric energy output from the energy output source 32 is lower in the second sealing mode than in the first sealing mode, the output control may be performed in a way other than the constant voltage control in each of the first and second sealing modes. For example, in a certain example, in the first sealing mode, the output controller 26 performs constant electric power control to maintain, with time, the output electric power P from the energy output source 32 at a constant level of first electric power P1. Further, in the second sealing mode, the output controller 26 performs constant electric power control to maintain, with time, the output electric power P from the energy output source 32 at a constant level of second electric power P2 lower than the first electric power P1. In another certain example, in the first sealing mode, it is possible to perform both the constant voltage control to maintain, with time, the output voltage V at a constant level of the first voltage value V1 and the constant electric power control to maintain, with time, the output electric power P at a constant level of the first electric power P1, and the switch is made between the constant voltage control and the constant electric power control in accordance with the impedance Z. Moreover, in the second sealing mode, it is possible to perform both the constant voltage control to maintain, with time, the output voltage V at a constant level of the second voltage value V2 lower than the first voltage value V1 and the constant electric power control to maintain, with time, the output electric power P at a constant level of the second electric power P2 lower than the first electric power PI, and the switch is made between the constant voltage control and the constant electric power control in accordance with the impedance Z. However, in each of the examples, the electric energy output from the energy output source 32 is lower in the second sealing mode than in the first sealing mode.

Furthermore, in the present embodiment, in each of the first and second sealing modes, the high-frequency current alone is applied to the blood vessel as the treatment energy, and treatment energy other than the high-frequency current, such as the ultrasonic vibration and the heater heat or the like, is not applied to the blood vessel (treated target). For example, in the example in which the ultrasonic transducer 46 is provided in the energy treatment instrument 2, the processor 21 stops the output of the electric energy to the ultrasonic transducer 46 from the energy output source 47 in each of the first and second sealing modes. Thus, in each of the first and second sealing modes, the electric energy is not supplied to the ultrasonic transducer 46, and no ultrasonic vibration is generated in the ultrasonic transducer 46. Similarly, in the example in which the heater is provided in the energy treatment instrument 2, the processor 21 stops the output of the electric energy to the heater from the energy output source in each of the first and second sealing modes. Thus, in each of the first and second sealing modes, the electric energy is not supplied to the heater, and no heat is generated in the heater.

In a certain example, if the output control in the first sealing mode and the output control in the second sealing mode are finished, no electric energy is supplied to the electrodes 27 and 28, the ultrasonic transducer 46, and the heater or the like, and treatment energy such as the high-frequency current, the ultrasonic vibration, and the heater heat or the like is not applied to the treated target. In another certain example, if the output control in the first sealing mode and the output control in the second sealing mode are finished, a shift is automatically made to output control for a cutting mode. In this case, in the example in which the ultrasonic transducer 46 is provided in the energy treatment instrument 2, the processor 21 causes the electric energy to be output to the ultrasonic transducer 46 from the energy output source 47 at a cutting level (high output level), in the cutting mode. Accordingly, ultrasonic vibration is generated in the ultrasonic transducer 46, and the ultrasonic vibration is transmitted to one of the grasping pieces 15 and 16. Then the transmitted ultrasonic vibration is applied to the grasped blood vessel (treated target) as the treatment energy, and the blood vessel is cut open by frictional heat resulting from the ultrasonic vibration. Similarly, in the example in which the heater is provided in the energy treatment instrument 2, the processor 21 causes the electric energy to be output to the heater from the energy output source at the cutting level (high output level), in the cutting mode. Accordingly, heat is generated in the heater. Then the heater heat is applied to the grasped blood vessel as the treatment energy, and the blood vessel is cut open.

FIG. 5 is a diagram showing one example of a change with time of the impedance Z between the pair of grasping pieces 15 and 16 (i.e., impedance of the grasped treated target) in a state where the processor 21 is performing output control in each of the first and second sealing modes. In FIG. 5, the impedance Z is indicated on the vertical axis, and the time t based on the start of the output of the electric energy from the energy output source 32 is indicated on the horizontal axis. In FIG. 5, the change with time of the impedance Z in the first sealing mode is indicated by a solid line, and the change with time of the impedance Z in the second sealing mode is indicated by a broken line. As shown in FIG. 5, when the output of the electric energy from the energy output source 32 is started and the high-frequency current starts flowing through the blood vessel (treated target), the impedance Z normally shows a behavior of decreasing with time for a while. Further, when the impedance Z decreases to some degree with time, the impedance Z normally shows a behavior of increasing with time in response to the increase of the temperature of the treated target due to the heat resulting from the high-frequency current.

In the present embodiment, as described above, the electric energy output from the energy output source 32 is lower in the second sealing mode than in the first sealing mode. Thus, a calorific value per unit time generated due to the high-frequency current flowing through the blood vessel (treated target) is lower in the second sealing mode than in the first sealing mode. Therefore, the increase rate of the temperature of the treated target (blood vessel) is lower, and the increase rate of the impedance Z in a state where the impedance Z increases with time is lower, in the second sealing mode than in the first sealing mode. Thus, the time for the impedance Z to reach the impedance threshold Zth1 from the start of the output of the electric energy from the energy output source 32 is longer in the second sealing mode than in the first sealing mode. Actually, in one example in FIG. 5, the impedance Z reaches the impedance threshold Zth1 at a time t1 in the first sealing mode, whereas the impedance Z reaches the impedance threshold Zth1 at a time t2 after the time t1 in the second sealing mode. In the present embodiment, as described above, in each of the first and second sealing modes, the output of the electric energy from the energy output source 32 is stopped on the basis of the fact that the impedance Z is equal to or more than the impedance threshold Zth1. Therefore, the output time of the electric energy from the energy output source 32 is longer in the second sealing mode than in the first sealing mode.

As described above, the output controller 26 (the processor 21) makes the electric energy output from the energy output source 32 lower and makes the output time of the electric energy from the energy output source 32 longer in the second sealing mode than in the first sealing mode. Thus, the calorific value per unit time generated due to the high-frequency current in the blood vessel is lower, and the time of the application of the high-frequency current to the blood vessel is longer in the second sealing mode than in the first sealing mode. That is, in the energy treatment instrument 2, the time of the application of the treatment energy (high-frequency current) to the treated target (blood vessel) from the energy application section (the grasping pieces 15 and 16) is longer in the second mode (second actuation mode) than in the first mode (first actuation mode). The magnitude of the total quantity of the treatment energy (high-frequency current) applied to the treated target in the first sealing mode corresponds to, for example, the magnitude of the area between the impedance Z and the time t indicated by the solid line in FIG. 5. Moreover, the magnitude of the total quantity of the treatment energy (high-frequency current) applied to the treated target in the second sealing mode corresponds to, for example, the magnitude of the area between the impedance Z and the time t indicated by the broken line in FIG. 5. Here, in FIG. 5, the area under the impedance Z in the second sealing mode indicated by the broken line is larger than the area under the impedance Z in the first sealing mode indicated by the solid line. Therefore, the performance of sealing the blood vessel by the high-frequency current is higher in the second sealing mode than in the first sealing mode.

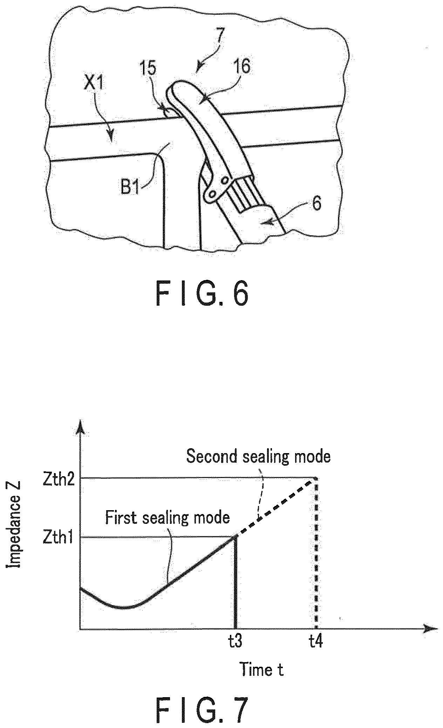

FIG. 6 is a diagram showing one example of an observation image generated by the processor 72 in a state where a blood vessel X1 is grasped between the grasping pieces 15 and 16. When the blood vessel X1 is grasped, a branched portion (a branch or the vicinity of the branch) B1 of the blood vessel X1 may be grasped as shown in FIG. 6 in some cases. Here, it is considered that in the blood vessel, the thickness of a blood vessel wall is larger in the branched portion than in portions that are not branched portions (portions with no branches present in the vicinity). Thus, there is concern that the treatment of sealing the blood vessel X1 using treatment energy such as the high-frequency current may be affected if the branched portion B1 of the blood vessel X1 is treated as in the case where the portions located apart from the branched portion B1 (the portions with no branches present in the vicinity) are sealed. Accordingly, there is a possibility that performance of sealing the blood vessel X1, such as a pressure resistance value of the sealed blood vessel X1, may be affected.

In the present embodiment, the processor 21 judges whether or not any branch of the blood vessel is present within the predetermined range (the range which is at the predetermined distance Lth or less) from the position where the blood vessel is grasped (the grasping position of the blood vessel) in the observation image. When it is judged that the branch of the blood vessel is not present within the predetermined range, the output control is performed in the first sealing mode, whereas when it is judged that a branch of the blood vessel is present within the predetermined range, the output control is performed in the second sealing mode. Thus, the electric energy output from the energy output source 32 is lower and the output time of the electric energy from the energy output source 32 is longer when it is judged that a branch of the blood vessel is present than when it is judged that the branch of the blood vessel is not present. That is, in the energy treatment instrument 2, the time of the application of the treatment energy (high-frequency current) to the treated target (blood vessel) from the energy application section (the grasping pieces 15 and 16) is longer in the second mode (second actuation mode) in the case where it is judged that a branch of the blood vessel is present within the predetermined range from the position where the blood vessel is grasped than in the first mode (first actuation mode) in the case where it is judged that the branch of the blood vessel is not present within the predetermined range. Therefore, when the blood vessel is grasped in the branched portion, the treatment is conducted in the second sealing mode in which the performance of sealing the blood vessel by the high-frequency current of the energy treatment instrument 2 of the treatment system 1 is higher than that in the first sealing mode, so that the blood vessel is sealed at the same level as in the case where the blood vessel is grasped in a portion located apart from the branch. Consequently, by the use of the energy treatment instrument 2 of the treatment system 1, performance of sealing the blood vessel, such as a pressure resistance value (difficulty of the flow of blood to the sealed portion) of the sealed blood vessel, is easily maintained when the blood vessel is grasped in the branched portion as well.

As described above, in the present embodiment, even when a branch of the blood vessel is present within the predetermined range from the position where the blood vessel is grasped (the grasping position of the blood vessel), the grasped blood vessel is properly sealed by the increase of the performance of sealing the blood vessel using the high-frequency current. That is, even if the blood vessel is grasped in the branched portion, the blood vessel X1 is properly sealed by use of treatment energy such as the high-frequency current, and suitable treatment performance (sealing performance) is achieved.

### (Modification of First Embodiment)

Note that in a first modification of the first embodiment, processing by the processor 21 in the output control in the second sealing mode is different from that in the first embodiment. In the present modification as well, the processor 21 performs processing similar to that in the first embodiment in the output control in the first sealing mode (see FIG. 4). In the output control in the second sealing mode as well as in the output control in the first sealing mode, the processor 21 performs processing in steps Sill to S113. However, in the second sealing mode, instead of the processing in step S114, the processor 21 judges whether or not the detected impedance Z is equal to or more than an impedance threshold (second impedance threshold) Zth2. Here, the impedance threshold Zth2 is higher than the impedance threshold (first impedance threshold) Zth1. Moreover, the impedance threshold Zth2 may be set by the surgeon or the like, or may be stored in the storage medium 22.

Furthermore, when the impedance Z is lower than the impedance threshold Zth2, the processing returns to step S112, and the processing in and after step S112 is sequentially performed. When the impedance Z is equal to or more than the impedance threshold Zth2, the output controller 26 stops the output of the electric energy (high-frequency electric power) from the energy output source 32. Therefore, in the second sealing mode according to the present modification, the output of the electric energy from the energy output source 32 is stopped on the basis of the fact that the impedance Z is equal to or more than the impedance threshold (second impedance threshold) Zth2 higher than the impedance threshold (first impedance threshold) Zth1. In the present modification as well, the processor 21 switches the actuation state of the energy treatment instrument 2 between the first mode (first actuation mode) and the second mode (second actuation mode) by controlling the output of the electric energy from the energy output source 32 on the basis of the judgement result of the branch of the blood vessel. Moreover, in the present modification as well, the output state of the electric energy from the energy output source 32 varies between the first sealing mode and the second sealing mode, so that in the energy treatment instrument 2, the state of the application of the treatment energy (high-frequency current) to the grasped treated target (blood vessel) from the energy application section (the grasping pieces 15 and 16) varies between the first mode and the second mode.

FIG. 7 is a diagram showing one example of a change with time of the impedance Z between the pair of grasping pieces 15 and 16 in a state where the processor 21 according to the present modification is performing output control in each of the first and second sealing modes. In FIG. 7, the impedance Z is indicated on the vertical axis, and the time t based on the start of the output of the electric energy from the energy output source 32 is indicated on the horizontal axis. In FIG. 7, the change with time of the impedance Z in the first sealing mode is indicated by a solid line, and the change with time of the impedance Z in the second sealing mode is indicated by a broken line.

As described above, in the present modification, the output of the electric energy from the energy output source 32 is stopped on the basis of the fact that the impedance Z is equal to or more than the impedance threshold Zth1 in the first sealing mode, whereas the output of the electric energy from the energy output source 32 is stopped on the basis of the fact that the impedance Z is equal to or more than the impedance threshold Zth2 in the second sealing mode. Moreover, the impedance threshold Zth2 is higher than the impedance threshold Zth1. Thus, the output time of the electric energy from the energy output source 32 is longer in the second sealing mode than in the first sealing mode. Actually, in one example in FIG. 7, the output of the electric energy is stopped at a time t3 in the first sealing mode, whereas the output of the electric energy is stopped at a time t4 after the time t3 in the second sealing mode.

As described above, in the present modification, the output controller 26 (the processor 21) sets a higher impedance threshold (Zth1; Zth2) to be the reference to stop the output in the second sealing mode than in the first sealing mode so that the output time of the electric energy from the energy output source 32 is longer in the second sealing mode than in the first sealing mode. That is, in the energy treatment instrument 2 according to the present modification as well, the time of the application of the treatment energy (high-frequency current) to the treated target (blood vessel) from the energy application section (the grasping pieces 15 and 16) is longer in the second mode (second actuation mode) in the case where it is judged that a branch of the blood vessel is present within the predetermined range from the position where the blood vessel is grasped than in the first mode (first actuation mode) in the case where it is judged that the branch of the blood vessel is not present within the predetermined range. Thus, the time of the application of the high-frequency current to the blood vessel is longer, and the total quantity of the treatment energy (high-frequency current) applied to the blood vessel is greater, so that the performance of sealing the blood vessel by the high-frequency current is higher in the second sealing mode than in the first sealing mode. Therefore, in the present modification as well, when the blood vessel is grasped in the branched portion (the branch and its vicinity), the treatment is conducted in the second sealing mode in which the performance of sealing the blood vessel by the high-frequency current of the energy treatment instrument 2 of the treatment system 1 is higher than that in the first sealing mode, so that the blood vessel is sealed at the same level as in the case where the blood vessel is grasped in a portion located apart from the branch. Consequently, by the use of the energy treatment instrument 2 of the treatment system 1, performance of sealing the blood vessel, such as a pressure resistance value (difficulty of the flow of blood to the sealed portion) of the sealed blood vessel, is easily maintained when the blood vessel is grasped in the branched portion as well.

Note that in a certain modification, the first embodiment may be combined with its first modification. In this case, the processor 21 makes the electric energy output from the energy output source 32 lower and sets a higher impedance threshold (Zth1; Zth2) to be the reference to stop the output in the second sealing mode than in the first sealing mode. In the present modification as well, the output state of the electric energy from the energy output source 32 varies between the first sealing mode and the second sealing mode, so that in the energy treatment instrument 2, the state of the application of the treatment energy (high-frequency current) to the grasped treated target (blood vessel) from the energy application section (the grasping pieces 15 and 16) varies between the first mode and the second mode.

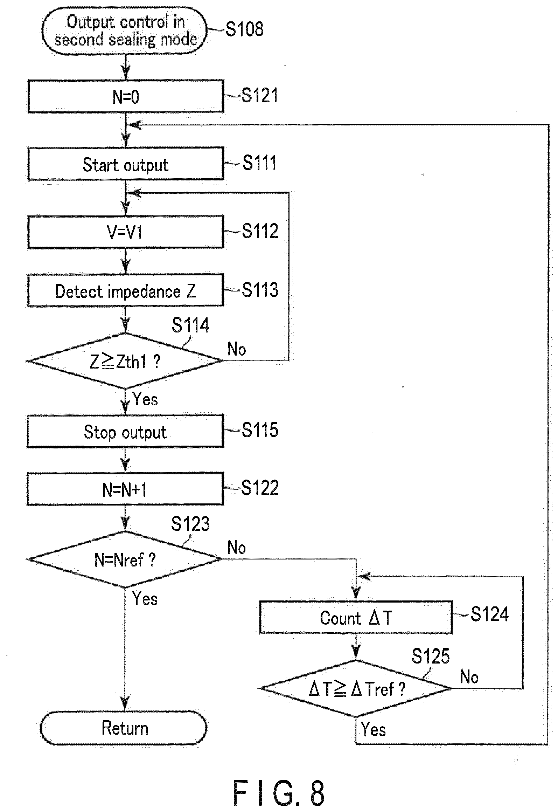

Furthermore, in the second modification of the first embodiment, the processor 21 performs processing shown in FIG. 8 in the output control in the second sealing mode. In the present modification as well, the processor 21 performs processing similar to that in the first embodiment in the output control in the first sealing mode (see FIG. 4). In the present modification, in the output control in the second sealing mode, the number of outputs N of the electric energy from the energy output source 32 is defined as a parameter. In the output control in the second sealing mode, the processor 21 sets the number of outputs N at 0 as an initial value (step S121). Further, as in the output control in the first sealing mode, the processor 21 performs the processing in steps Sill to S115.

If the output of the electric energy from the energy output source 32 is stopped by the processing in step S115, the processor 21 adds one to the number of outputs N (step S122). Then the processor 21 judges whether or not the number of outputs N after the addition is the same as a reference number of times Nref (step S123). The reference number of times Nref is a natural number of 2 or more, and may be set by the surgeon or the like, or may be stored in the storage medium 22. When the number of outputs N is the same as the reference number of times Nref, that is, when the number of outputs N has reached the reference number of times Nref (step S123 - Yes), the processor 21 finishes the output control in the second sealing mode. Consequently, for example, the output of the electric energy from the energy output source 32 is continuously kept stopped.

Here, a time (elapsed time) ΔT at which a point nearest the point where the output of the electric energy from the energy output source 32 is stopped by the processing in step S115 is 0 is defined. When the number of outputs N is not the same as the reference number of times Nref, that is, when the number of outputs N has not reached the reference number of times Nref (step S123 - No), the processor 21 counts the time ΔT (step S124). Then the processor 21 judges whether or not the time ΔT that is being counted is equal to or more than a reference time ΔTref (step S125). The reference time ΔTref is, for example, 10 msec, and may be set by the surgeon or the like, or may be stored in the storage medium 22.

When the time ΔT is shorter than the reference time ΔTref (step S125 - No), the processing returns to step S124, and the processing in and after step S124 is sequentially performed. That is, the output of the electric energy from the energy output source 32 is kept stopped, and the time ΔT is continuously counted. When the time ΔT is equal to or more than the reference time ΔTref (step S125 - Yes), the processing returns to step Sill, and the processing in and after step Sill is sequentially performed. That is, the electric energy is again output from the energy output source 32.

The processing described above is performed, so that in the output control in the second sealing mode, the output controller 26 of the processor 21 stops the output of the electric energy from the energy output source 32 after starting the output of the electric energy from the energy output source 32, and again starts the output of the electric energy from the energy output source 32 after once stopping the output of the electric energy from the energy output source 32. That is, in the second sealing mode, the electric energy is again output from the energy output source 32 when the reference time ΔTref elapses from the point where the output of the electric energy from the energy output source 32 is once stopped. Moreover, in the output control in the second sealing mode, the processor 21 causes the electric energy to be intermittently output from the energy output source 32 the reference number of times Nref (more than one time). In the present modification as well, the processor 21 switches the actuation state of the energy treatment instrument 2 between the first mode (first actuation mode) and the second mode (second actuation mode) by controlling the output of the electric energy from the energy output source 32 on the basis of the judgement result of the branch of the blood vessel. In the present modification as well, the output state of the electric energy from the energy output source 32 varies between the first sealing mode and the second sealing mode, so that in the energy treatment instrument 2, the state of the application of the treatment energy (high-frequency current) to the grasped treated target (blood vessel) from the energy application section (the grasping pieces 15 and 16) varies between the first mode and the second mode.

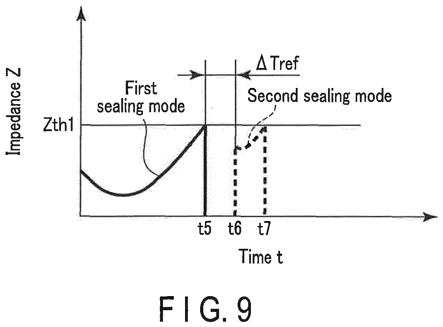

FIG. 9 is a diagram showing one example of a change with time of the impedance Z between the pair of grasping pieces 15 and 16 in a state where the processor 21 according to the present modification is performing output control in each of the first and second sealing modes. In FIG. 9, the impedance Z is indicated on the vertical axis, and the time t based on the start of the output of the electric energy from the energy output source 32 is indicated on the horizontal axis. In FIG. 9, the change with time of the impedance Z in the first sealing mode is indicated by a solid line, and the change with time of the impedance Z in the second sealing mode is indicated by a broken line. In one example shown in FIG. 9, in each of the first and second sealing modes, the output of the electric energy from the energy output source 32 is stopped at a time t5 on the basis of the fact that the impedance Z has reached the impedance threshold Zth1.

As described above, in the present modification, the electric energy is intermittently output from the energy output source 32 more than one time (the reference number of times Nref) in the second sealing mode. Thus, in one example shown in FIG. 9, in the second sealing mode, the output of the electric energy from the energy output source 32 is again started at a time t6 at which the reference time ΔTref elapses from the time t5 when the output is stopped. In this instance, the impedance Z is lower than the impedance threshold Zth1. Further, at a time t7 after the time t6 (the time at which the output of the electric energy is again started), the output of the electric energy from the energy output source 32 is again stopped on the basis of the fact that the impedance Z has reached the impedance threshold Zth1. Note that the reference number of times Nref is 2 in the example in FIG. 9.

As described above, in the present modification, the output controller 26 (the processor 21) again starts the output of the electric energy after once stopping the output, in the second sealing mode. Thus, the output time of the electric energy from the energy output source 32 is longer, and the time of the application of the high-frequency current to the blood vessel is longer in the second sealing mode than in the first sealing mode. That is, in the energy treatment instrument 2 according to the present modification as well, the time of the application of the treatment energy (high-frequency current) to the treated target (blood vessel) from the energy application section (the grasping pieces 15 and 16) is longer in the second mode (second actuation mode) in the case where it is judged that a branch is present within the predetermined range from the position where the treated target (blood vessel) is grasped than in the first mode (first actuation mode) in the case where it is judged that the branch is not present within the predetermined range. Thus, the performance of sealing the blood vessel by the high-frequency current is higher in the second sealing mode than in the first sealing mode. Therefore, in the present modification as well, when the blood vessel is grasped in the branched portion (the branch and its vicinity), the treatment is conducted in the second sealing mode in which the performance of sealing the blood vessel by the high-frequency current of the energy treatment instrument 2 of the treatment system 1 is higher than that in the first sealing mode, so that the blood vessel is sealed at the same level as in the case where the blood vessel is grasped in a portion located apart from the branch. Consequently, by the use of the energy treatment instrument 2 of the treatment system 1, performance of sealing the blood vessel, such as a pressure resistance value (difficulty of the flow of blood to the sealed portion) of the sealed blood vessel, is easily maintained when the blood vessel is grasped in the branched portion as well.

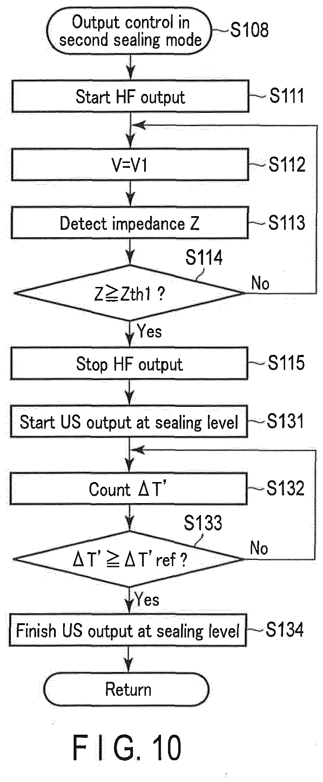

Furthermore, in a third modification of the first embodiment, the processor 21 performs the processing shown in FIG. 10 in the output control in the second sealing mode. In the present modification as well, the processor 21 performs processing similar to that in the first embodiment in the output control in the first sealing mode (see FIG. 4). Further, in the output control in the second sealing mode as well as in the output control in the first sealing mode, the processor 21 performs the processing in steps Sill to S115.

In the second sealing mode, when the output of the electric energy from the energy output source 32 is stopped by the processing in step S115, the output controller 26 of the processor 21 starts the output of the electric energy to the ultrasonic transducer 46 from the energy output source 47 (step S131). In this instance, the electric energy is output at a sealing level of a low output level in the energy output source 47. That is, the output level is lower in the output of the electric energy at the sealing level than in the output of the electric energy at the cutting level described above. Thus, the electric energy supplied to the ultrasonic transducer 46 is lower, and the amplitude of the ultrasonic vibration transmitted to one of the grasping pieces 15 and 16 is lower in the output at the sealing level than in the output at the cutting level. Therefore, in the output at the sealing level, the calorific value of the frictional heat resulting from the ultrasonic vibration is low, the grasped blood vessel is not cut open by the frictional heat, and the blood vessel is only sealed. Note that in FIG. 10, the output of the electric energy to the electrodes 27 and 28 from the energy output source 32 is indicated as high-frequency (HF) output, and the output of the electric energy to the ultrasonic transducer 46 from the energy output source 47 is indicated as ultrasonic (US) output.

Here, a time (elapsed time) ΔT' at which a point where the output of the electric energy from the energy output source 47 at the sealing level is started by the processing in step S131 (a point where the output from the energy output source 32 is stopped by the processing in step S115) is 0 is defined. When the output of the electric energy from the energy output source 47 at the sealing level is started, the processor 21 counts the time ΔT' (step S132). Then the processor 21 judges whether or not the time ΔT' that is being counted is equal to or more than a reference time ΔT'ref (step S133). The reference time ΔT'ref may be set by the surgeon or the like, or may be stored in the storage medium 22.

When the time ΔT' is shorter than the reference time ΔT'ref (step S133 - No), the processing returns to step S132, and the processing in and after step S132 is sequentially performed. That is, the time ΔT' is continuously counted. When the time ΔT' is equal to or more than the reference time ΔT'ref (step S133 - Yes), the output controller 26 finishes the output of the electric energy from the energy output source 47 at the sealing level (step S134). In this instance, the output of the electric energy to the ultrasonic transducer 46 from the energy output source 47 may be stopped, a shift may be automatically made to the output control in the cutting mode, and a switch may be automatically made so that the electric energy is output to the ultrasonic transducer 46 at the cutting level (high output level). Moreover, in a certain example, the output controller 26 may finish the output of the electric energy from the energy output source 47 at the sealing level on the basis of the fact that the operation input with the operational button (energy operation input section) 18 is cancelled (i.e., the fact that the operation input is turned off), instead of steps S132 and S133.

As described above, in the present modification, the output controller 26 (the processor 21) starts the output of the electric energy to the ultrasonic transducer 46 after stopping the output of the electric energy to the electrodes 27 and 28, in the second sealing mode. That is, the processor 21 switches the actuation state of the energy treatment instrument 2 between the first mode (first actuation mode) and the second mode (second actuation mode) by controlling the output of the electric energy from the energy output sources 32 and 47 on the basis of the judgement result of the branch of the blood vessel. Moreover, in the present modification, the electric energy is output from the energy output source 47 in the second sealing mode alone, so that in the energy treatment instrument 2, the state of the application of the treatment energy (high-frequency current and ultrasonic vibration) to the grasped treated target (blood vessel) from the energy application section (the grasping pieces 15 and 16) varies between the first mode and the second mode. Thus, in the second sealing mode, the grasped blood vessel is sealed by the ultrasonic vibration (frictional heat) even after the output of the electric energy to the electrodes 27 and 28 is stopped. That is, in the second sealing mode, the impedance Z is higher, so that the blood vessel is sealed by the frictional heat resulting from the ultrasonic vibration even in a state where it is difficult for the high-frequency current to flow through the blood vessel. Thus, the performance of sealing the blood vessel by the treatment energy is higher in the second sealing mode than in the first sealing mode. Therefore, in the present modification as well, when the blood vessel is grasped in the branched portion (the branch and its vicinity), the treatment is conducted in the second sealing mode in which the performance of sealing the blood vessel by the treatment energy of the energy treatment instrument 2 of the treatment system 1 is higher than that in the first sealing mode, so that the blood vessel is sealed at the same level as in the case where the blood vessel is grasped in a portion located apart from the branch. Consequently, by the use of the energy treatment instrument 2 of the treatment system 1, performance of sealing the blood vessel, such as a pressure resistance value (difficulty of the flow of blood to the sealed portion) of the sealed blood vessel, is easily maintained when the blood vessel is grasped in the branched portion as well.

Note that in a certain modification, in the second sealing mode, when the output of the electric energy from the energy output source 32 is stopped by the processing in step S115, the output controller 26 of the processor 21 starts the output of the electric energy to the heater. In this instance as well, the electric energy is output at the sealing level which is lower in the output level than the cutting level described above. Thus, the electric energy supplied to the heater is lower in the output at the sealing level than in the output at the cutting level. Therefore, in the output at the sealing level, the calorific value of the heat generated in the heater is lower, the grasped blood vessel is not cut open by the heater heat, and the blood vessel is only sealed. In the present modification, the blood vessel is sealed by the heater heat in addition to the high-frequency current in the second sealing mode. That is, in the present modification as well, in the energy treatment instrument 2, the state of the application of the treatment energy (high-frequency current and heater heat) to the grasped treated target (blood vessel) from the energy application section (the grasping pieces 15 and 16) varies between the first mode and the second mode. Therefore, the performance of sealing the blood vessel by the treatment energy is higher in the second sealing mode than in the first sealing mode. Thus, functions and advantageous effects similar to those in the third modification of the first embodiment are provided.

Furthermore, the output control of the electric energy in which the performance of sealing the blood vessel by the treatment energy is higher when it is judged that a branch of the blood vessel is present within the predetermined range from the grasping position than when it is judged that the branch of the blood vessel is not present within the predetermined range is also applicable to an example in which no high-frequency current is applied to the blood vessel and in which the treatment energy (ultrasonic vibration and heater heat or the like) other than the high-frequency current is only applied to the blood vessel. For example, in a certain modification in which the electric energy is output to the ultrasonic transducer 46 at the sealing level and the blood vessel is sealed by use of the ultrasonic vibration alone, the processor 21 makes the electric energy output to the ultrasonic transducer 46 from the energy output source 47 lower and makes the output time of the electric energy to the ultrasonic transducer 46 longer in the second sealing mode (the second mode of the energy treatment instrument 2) than in the first sealing mode (the first mode of the energy treatment instrument 2). Thus, the time of the application of the ultrasonic vibration to the blood vessel is longer, and the performance of sealing the blood vessel by the ultrasonic vibration is higher in the second sealing mode (when it is judged that a branch of the blood vessel is present) than in the first sealing mode (when it is judged that the branch of the blood vessel is not present). Moreover, in a certain modification in which the electric energy is output to the heater at the sealing level and the blood vessel is sealed by the heater heat alone, the processor 21 makes-the electric energy output to the heater from the energy output source lower and makes the output time of the electric energy to the heater longer in the second sealing mode than in the first sealing mode. Thus, the time of the application of the heater heat to the blood vessel is longer, and the performance of sealing the blood vessel by the heater heat is higher in the second sealing mode (when it is judged that a branch of the blood vessel is present) than in the first sealing mode (when it is judged that the branch of the blood vessel is not present). Consequently, by the use of the energy treatment instrument 2 of the treatment system 1, performance of sealing the blood vessel, such as a pressure resistance value (difficulty of the flow of blood to the sealed portion) of the sealed blood vessel, is easily maintained when the blood vessel is grasped in the branched portion (the branch and its vicinity) as well.

Furthermore, in a certain modification, the surgeon or the like may judge whether to cause the processor 21 to perform the output control in the first sealing mode or perform the output control in the second sealing mode. In the present modification, two operational buttons or the like which are energy operation input sections are provided, and if an operation input is performed with one of the operational buttons, the processor 21 (the output controller 26) performs the output control of the electric energy in the first sealing mode, and the energy treatment instrument 2 is actuated in the first mode (first actuation mode) to coagulate the treated target (blood vessel). Then, when an operation input is performed with the other of the operational buttons, the processor 21 performs the output control of the electric energy in the second sealing mode in which the performance of sealing the blood vessel by the treatment energy is higher than that in the first sealing mode. In the present modification, a notification section (not shown) which shows a judgement result of whether or not a branch of the blood vessel is present within the predetermined range from the position where the blood vessel is grasped (the grasping position of the blood vessel) is provided in, for example, the controller 3. In a certain example, the notification section is an LED, and lights when it is judged that a branch of the blood vessel is present within the predetermined range. In another example, the notification section may be a buzzer, a display screen, or the like.

Furthermore, in another certain modification, the display device 67 functions as the notification section, and at least one of the observation image and the result of the image processing is displayed on the display device 67. In the present modification, the surgeon judges whether or not a branch of the blood vessel is present within the predetermined range from the position where the blood vessel is grasped (the grasping position of the blood vessel), on the basis of the observation image and/or the result of the image processing displayed on the display device 67. Then the surgeon judges which of the two operational buttons is used to perform an operation input, and selects whether to cause the processor 21 to perform the output control in the first sealing mode or perform the output control in the second sealing mode.

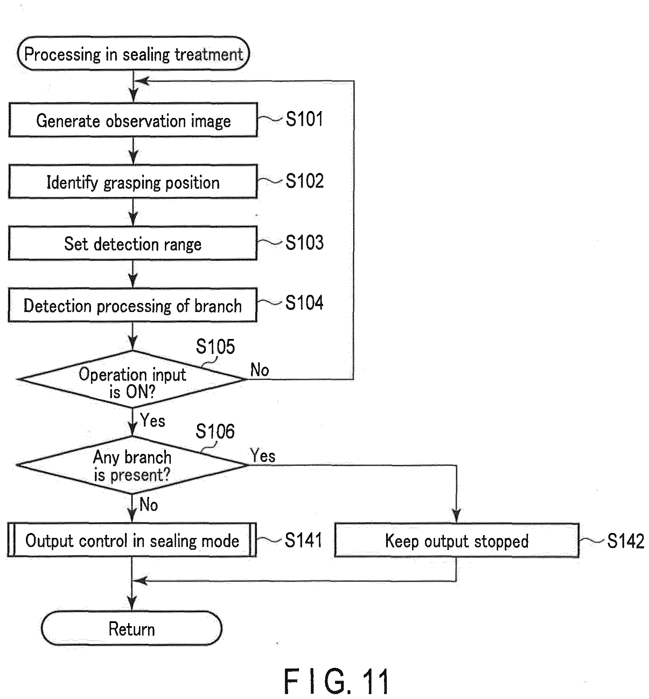

Furthermore, in a fourth modification of the first embodiment, the processors 21 and 72 perform the processing shown in FIG. 11 in the sealing treatment of the blood vessel. In the present modification as well as in the embodiment described above, the processors 21 and 72 perform the processing in steps S101 to S106 in the sealing treatment of the blood vessel. Further, when it is judged that the branch of the blood vessel is not present within the predetermined range from the grasping position of the blood vessel (step S106 - No), the processor 21 performs the output control of the electric energy in the sealing mode (step S141). In the output control in the sealing mode, the processor 21 performs, for example, processing similar to that in the output control in the first sealing mode according to the first embodiment (see FIG. 4). The processor 21 performs the output control of the electric energy in the first sealing mode, and the energy treatment instrument 2 is thereby actuated in the first mode to coagulate the grasped blood vessel. When it is judged that a branch of the blood vessel is present within the predetermined range (step S106 - Yes), the processor 21 keeps the output of the electric energy stopped regardless of the presence of an operation input with the operational button 18 (step S142). In this instance, the energy treatment instrument 2 is actuated in the second mode. That is, the output of the electric energy from the energy output sources 32 and 47 or the like is kept stopped. Thus, when it is judged that a branch of the blood vessel is present, no treatment energy such as the high-frequency current is applied to the grasped blood vessel even if an operation input is performed with the operational button 18. Therefore, in the present modification as well, the processor 21 switches the actuation state of the energy treatment instrument 2 between the first mode (first actuation mode) and the second mode (second actuation mode) by controlling the output of the electric energy from the energy output source 32 on the basis of the judgement result of the branch of the blood vessel. In the present modification, the output of the electric energy from the energy output sources 32 and 47 or the like is stopped in the second mode, so that in the energy treatment instrument 2, the state of the application of the treatment energy (high-frequency current or the like) to the grasped treated target (blood vessel) from the energy application section (the grasping pieces 15 and 16) varies between the first mode and the second mode.

The output control is performed as described above, so that in the present modification, no treatment energy is applied to the blood vessel when a branched point of the blood vessel is present within the predetermined range from the position where the blood vessel is grasped (the grasping position of the blood vessel). That is, no treatment energy is applied to the blood vessel in a state where the sealing performance is affected, for example, when the blood vessel is grasped in the branched portion. Because the treatment energy is applied to the blood vessel only in a state where the effect on the sealing performance is small, for example, when the blood vessel is grasped in a portion located apart from the branched portion, the blood vessel is properly sealed by use of the treatment energy such as the high-frequency current, and suitable treatment performance (sealing performance) is achieved.

Furthermore, in a certain modification, the surgeon or the like may judge whether or not to output the electric energy in the sealing mode. In the present modification, the aforementioned notification section is provided in, for example, the controller 3. When it is notified or judged that the branch of the blood vessel is not present within the predetermined range from the grasping position of the blood vessel, the surgeon performs an operation input with the operational button 18, and causes the processor 21 to perform the output control in the sealing mode. Accordingly, the electric energy is output from the energy output sources 32 and 47 or the like, and the energy treatment instrument 2 is actuated in the first mode (first actuation mode). On the other hand, when it is notified or judged that a branch of the blood vessel is present within the predetermined range, the surgeon does not perform an operation input with the operational button 18. Thus, no electric energy is output from the energy output sources 32 and 47 or the like, and the energy treatment instrument 2 is actuated in the second mode (second actuation mode) different from the first mode.

### (Second Embodiment)

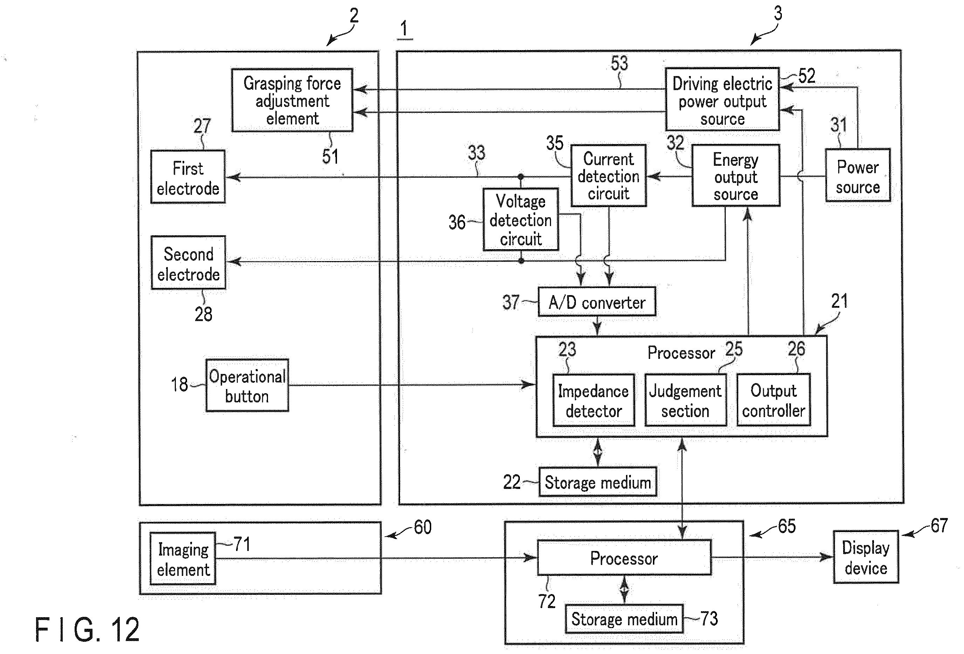

Next, a second embodiment of the present invention is described with reference to FIG. 12 to FIG. 14. The second embodiment is a modification in which the configuration according to the first embodiment is modified as below. Note that the same parts as those in the first embodiment are denoted with the same reference signs and are not described.

FIG. 12 is a diagram showing a control configuration in the treatment system 1 according to the present embodiment. As shown in FIG. 12, in the present embodiment, a grasping force adjustment element 51 is provided in the energy treatment instrument 2. The force of grasping the treated target (blood vessel) between the grasping pieces 15 and 16 changes in accordance with the driving state of the grasping force adjustment element 51. That is, the force of grasping the treated target between the grasping pieces 15 and 16 is adjusted by the grasping force adjustment element 51. Moreover, in the present embodiment, a driving electric power output source 52 is provided in the controller 3. The driving electric power output source 52 is electrically connected to the grasping force adjustment element 51 via an electricity supply path 53 extending through the cable 10. Here, the driving electric power output source 52 may be integral with the aforementioned energy output sources 32 and 47 or the like, or may be formed separately from the energy output sources 32 and 47 or the like.

The driving electric power output source 52 comprises a conversion circuit, an amplifier circuit, and others, and converts electric power from the power source 31 into driving electric power for the grasping force adjustment element 51. Then the driving electric power output source 52 outputs the driving electric power resulting from the conversion, and the output driving electric power is supplied to the grasping force adjustment element 51 through the electricity supply path 53. The processor 21 controls driving of the driving electric power output source 52, and controls the output of the driving electric power from the driving electric power output source 52. Thereby, the supply of the driving electric power to the grasping force adjustment element 51 is controlled, and the driving of the grasping force adjustment element 51 is controlled. In the present embodiment, the actuation state of the energy treatment instrument 2 is switched between the first mode (first actuation mode) and the second mode (second actuation mode) in accordance with the driving state of the grasping force adjustment element 51. In the present embodiment, the force of grasping the treated target (blood vessel) between the grasping pieces 15 and 16 varies between the first mode and the second mode.

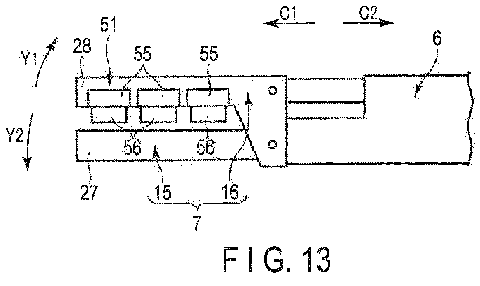

FIG. 13 is a diagram showing one example of the grasping force adjustment element 51. In the example shown in FIG. 13, a heater 55 and a volume changing portion 56 are provided as the grasping force adjustment element 51 in the second grasping piece 16. The volume changing portion 56 is made of an electrically insulating material such as parylene, nylon, or ceramics, and can abut on the first grasping piece 15 (the first electrode 27) by the closing of the grasping pieces 15 and 16. In a state where the volume changing portion 56 is in abutment with the first grasping piece 15, the electrodes 27 and 28 are apart from each other, and the contact between the electrodes 27 and 28 is prevented by the volume changing portion 56. Moreover, the volume changing portion 56 is made of a material having a high thermal expansion coefficient.

The driving electric power is output to the heater 55 from the driving electric power output source 52, whereby the grasping force adjustment element 51 is driven, and heat is generated in the heater 55. Due to the heat generated in the heater 55, the temperature of the volume changing portion 56 rises, and the volume changing portion 56 expands (the volume of the volume changing portion 56 increases.). By the expansion of the volume changing portion 56 in a state where the blood vessel (treated target) is grasped between the grasping pieces 15 and 16, the distance between the grasping pieces 15 and 16 decreases, and the force of grasping the treated target between the grasping pieces 15 and 16 increases. Note that in the present example, the coagulation and cutting or the like of the treated target are not performed by the heat generated in the heater 55.

Furthermore, in another certain example, a Peltier element may be provided instead of the heater 55. In this case, the driving electric power is output to the Peltier element from the driving electric power output source 52, and the Peltier element thereby moves the heat to the side of the volume changing portion 56. Due to the movement of the heat by the Peltier element, the temperature of the volume changing portion 56 rises, and the volume changing portion 56 expands. Thus, in a state where the blood vessel (treated target) is grasped between the grasping pieces 15 and 16, the distance between the grasping pieces 15 and 16 decreases, and the force of grasping the treated target between the grasping pieces 15 and 16 increases, as described above.

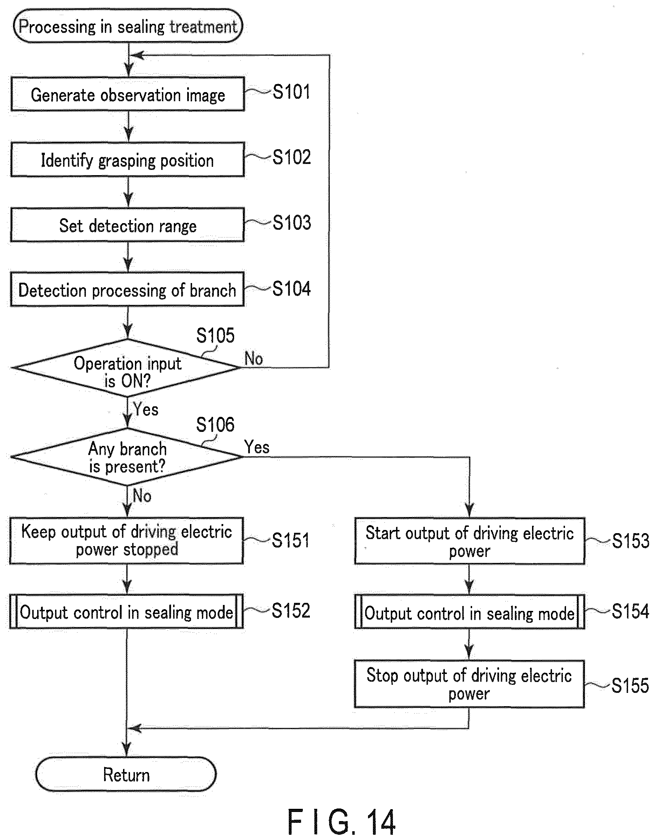

Now, functions and advantageous effects according to the present embodiment are described. FIG. 14 is a flowchart showing processing at the processors 21 and 72 in a sealing treatment of the blood vessel using the treatment system 1 according to the present embodiment. In the present embodiment as well as in the embodiment and others described above, the processor 21 performs the processing shown in steps S101 to S106 in the sealing treatment of the blood vessel. Further, when it is judged that the branch of the blood vessel is not present within the predetermined range from the grasping position of the blood vessel (step S106 - Yes), the processor 21 keeps the output of the driving electric power to the grasping force adjustment element 51 from the driving electric power output source 52 stopped (step S151). Thus, the grasping force adjustment element 51 is not driven, and the volume changing portion 56 does not expand. Therefore, the force of grasping the treated target between the grasping pieces 15 and 16 is maintained. Then the processor 21 performs the output control of the electric energy from the energy output source 32 or the like in the sealing mode (step S152). In the output control in the sealing mode, the processor 21 performs, for example, processing similar to that in the output control in the first sealing mode according to the first embodiment (see FIG. 4). In a state where the output of the driving electric power to the grasping force adjustment element 51 from the driving electric power output source 52 is stopped by the processor 21 and the grasping force adjustment element 51 is not driven, the energy treatment instrument 2 is actuated in the first mode (first actuation mode) to coagulate the grasped blood vessel.

On the other hand, when it is judged that a branch of the blood vessel is present within the predetermined range (step S106 - No), the processor 21 starts the output of the driving electric power to the grasping force adjustment element 51 from the driving electric power output source 52 (step S153). Thus, the grasping force adjustment element 51 is driven, and the volume changing portion 56 expands. Therefore, the force of grasping the treated target between the grasping pieces 15 and 16 increases. Then the processor 21 performs the output control of the electric energy from the energy output source 32 or the like in the sealing mode (step S154). In the output control in the sealing mode, the processor 21 performs, for example, processing similar to that in the output control in the first sealing mode according to the first embodiment (see FIG. 4). After finishing the output control in the sealing mode, the processor 21 stops the output of the driving electric power to the grasping force adjustment element 51 from the driving electric power output source 52 (step S155). In a state where the driving electric power is output to the grasping force adjustment element 51 from the driving electric power output source 52 by the processor 21 and the grasping force adjustment element 51 is driven, the energy treatment instrument 2 coagulates the grasped blood vessel, and is actuated in the second mode (second actuation mode) different from the first mode. As described above, in the present embodiment, the processor 21 switches the actuation state of the energy treatment instrument 2 between the first mode (first actuation mode) and the second mode (second actuation mode) by controlling the output of the driving electric power from the driving electric power output source 52 on the basis of the judgement result of the branch of the blood vessel. In the energy treatment instrument 2, the driving state of the grasping force adjustment element 51 varies between the first mode and the second mode, so that the force of grasping the treated target (blood vessel) between the grasping pieces 15 and 16 varies between the first mode and the second mode.

The control by the processor 21 is performed as described above, whereby in the present modification, the processor 21 makes the force of grasping the blood vessel (treated target) between the grasping pieces 15 and 16 greater when it is judged that a branch of the blood vessel is present within the predetermined range from the grasping position of the blood vessel than when it is judged that the branch of the blood vessel is not present within the predetermined range. That is, in the energy treatment instrument 2, the force of grasping the blood vessel (treated target) between the grasping pieces 15 and 16 is greater in the second mode (second actuation mode) than in the first mode (first actuation mode). Thus, even if a branch of the blood vessel is present within the predetermined range from the position where the blood vessel is grasped (the grasping position of the blood vessel), the force of grasping the blood vessel between the grasping pieces 15 and 16 is increased, so that the grasped blood vessel is properly sealed. That is, even if the blood vessel is grasped in the branched portion (the branch and its vicinity), the blood vessel is properly sealed by use of the treatment energy, and suitable treatment performance (sealing performance) is achieved.

### (Modification of Second Embodiment)

Note that the grasping force adjustment element 51 is not limited to the configuration described above. For example, in a certain modification, an electric motor and an abutment member are provided as the grasping force adjustment element 51. In this case, the handle 12 is closed relative to the grip 11, and the handle 12 thereby abuts on the abutment member, and the handle 12 closes relative to the grip 11 until abutting on the abutment member. Then the processor 21 (the output controller 26) controls the output of the driving electric power to the electric motor from the driving electric power output source 52, and controls the driving of the electric motor. Due to the driving of the electric motor, the abutment member moves, and the position of the abutment member changes. Accordingly, the stroke of the handle at the time of the closing of the handle 12 relative to the grip 11 changes. In the present modification, the processor 21 adjusts the position of the abutment member on the basis of a load **σ,** thereby making the stroke of the handle 12 at the time of its closing greater when it is judged that a branch of the blood vessel is present within the predetermined range from the grasping position of the blood vessel (the second mode of the energy treatment instrument 2) than when it is judged that the branch of the blood vessel is not present within the predetermined range (the first mode of the energy treatment instrument 2). Consequently, in the present modification as well, the force of grasping the blood vessel (treated target) between the grasping pieces 15 and 16 is greater when it is judged that a branch of the blood vessel is present within the predetermined range than when it is judged that the branch of the blood vessel is not present within the predetermined range.

Furthermore, according to the configuration in which one of the grasping pieces 15 and 16 is formed by the rod member that is inserted through the sheath 6, a support member which supports the rod member on the most distal side inside the sheath 6, and the electric motor or the like which is driven and thereby moves the support member are provided as the grasping force adjustment element 51. In this case, the electric motor or the like is driven in accordance with the judgement result regarding the branch, and thereby the position at which the rod member is supported by the support member is changed. Accordingly, in a state where the treated target (blood vessel) is grasped between the grasping pieces 15 and 16, the bending amount of a distal portion of the rod member (one of the grasping pieces 15 and 16) changes, and the grasping force between the grasping pieces 15 and 16 changes. Moreover, the control to adjust the grasping force as in the second embodiment is suitably applicable if the grasping force adjustment element 51 which changes the force of grasping the treated target (blood vessel) between the grasping pieces 15 and 16 is provided.

Furthermore, in another certain modification, an operational button or the like may be provided as a driving operation input section which outputs driving electric power from the driving electric power output source 52. In the present modification, the surgeon or the like judges whether or not to output the driving electric power. Moreover, in the present modification, the aforementioned notification section is provided in, for example, the controller 3 or the display device 67. When it is notified or judged that the branch of the blood vessel is not present within the predetermined range from the grasping position of the blood vessel, the surgeon does not perform an operation input with the operational button (driving operation input section). Thus, no driving electric power is output to the grasping force adjustment element 51 (the heater 55) from the driving electric power output source 52, and the volume changing portion 56 does not expand. Accordingly, the energy treatment instrument 2 is actuated in the first mode (first actuation mode). On the other hand, when it is notified or judged that a branch of the blood vessel is present within the predetermined range, the surgeon performs an operation input with the operational button 18. Thus, the driving electric power is output to the grasping force adjustment element 51 (the heater 55) from the driving electric power output source 52, and the volume changing portion 56 expands due to the heat generated in the heater 55. Accordingly, the energy treatment instrument 2 is actuated in the second mode (second actuation mode), and the force of grasping the treated target between the grasping pieces 15 and 16 increases.

### (Other Modifications)

Note that in a certain modification, one of the first embodiment and its modifications and one of the second embodiment and its modifications may be combined. In this case, when it is judged that the branch of the blood vessel is not present within the predetermined range from the grasping position of the blood vessel, the processor 21 performs the output control of the electric energy from the energy output sources 32 and 47 or the like in the first sealing mode, thereby applying the treatment energy to the blood vessel. Further, when it is judged that a branch of the blood vessel is present within the predetermined range, the processor 21 performs the output control of the electric energy from the energy output sources 32 and 47 or the like in the second sealing mode in which the performance of sealing the blood vessel by the treatment energy is higher than that in the first sealing mode, thereby applying the treatment energy to the blood vessel. That is, in the present modification as well as in the first embodiment, the performance of sealing the blood vessel by the treatment energy is higher in the second sealing mode of the energy treatment instrument 2 than in the first sealing mode. Moreover, in the present modification, the processor 21 makes the force of grasping the treated target between the grasping pieces 15 and 16 greater when it is judged that a branch of the blood vessel is present within the predetermined range (the second mode of the energy treatment instrument 2) than when it is judged that the branch of the blood vessel is not present within the predetermined range (the first mode of the energy treatment instrument 2).

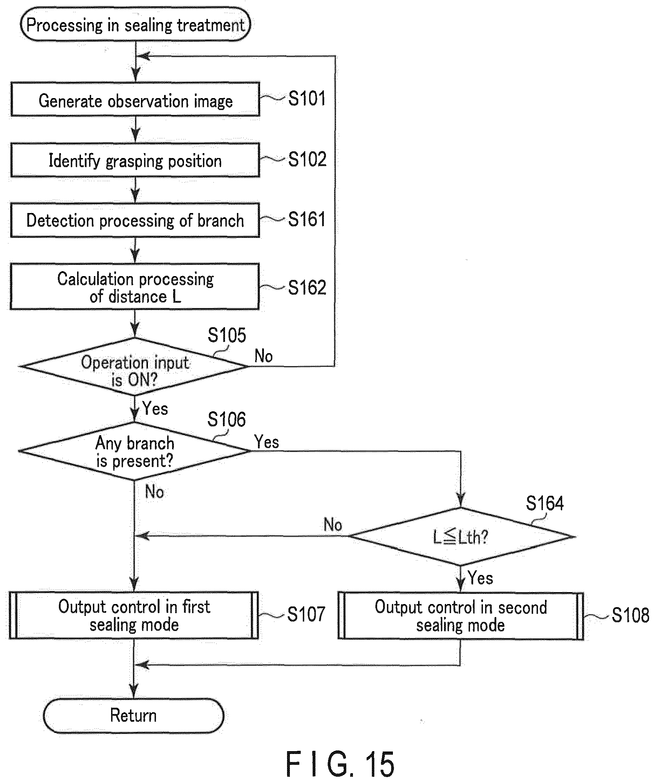

Furthermore, in a certain modification, as shown in FIG. 15, when the processor 72 identifies the position where the blood vessel is grasped in the observation image (step S102), the processor 72 performs, as image processing, the detection processing of the branch of the blood vessel in the whole observation image as a detection range (step S161). Then the processor 72 of the image processing device 65 identifies the position of the branch detected in the detection processing of the branch of the blood vessel, and performs calculation processing of a distance L between the detected branch and the grasping position of the blood vessel (step S162). Note that in the present modification as well, the detection processing of the branch is performed as in, for example, Japanese Patent No. 2011-167529, as described above. Moreover, in the present modification as well, when the operation input is not performed (step S105 - No), the processing returns to step S101, and the processing in and after step S101 is sequentially performed. Thus, the generation of the observation image and the detection processing of the branch of the blood vessel in the whole observation image are repeated.

Furthermore, when the operation input is performed (step S105 - Yes), the judgement section 25 of the processor 21 judges whether or not any branch of the blood vessel is present regarding the whole observation image, on the basis of the detection result in the detection processing of the branch of the blood vessel (step S163). When it is judged that the branch of the blood vessel is not present (step S163 - No), the processor 21 performs, for example, the output control in the first sealing mode (step S107). On the other hand, when it is judged that a branch of the blood vessel is present (step S163 - Yes), the judgement section 25 judges whether or not the calculated distance L is less than or equal to the predetermined distance Lth, on the basis of the calculation result in the calculation processing of the distance L between the detected branch and the grasping position of the blood vessel (step S164). The predetermined distance L is stored in, for example, the storage medium 22. When the distance L is greater than the predetermined distance Lth (step S164 - No), the processor 21 performs, for example, the output control in the first sealing mode (step S107). On the other hand, when the distance L is less than or equal to the predetermined distance Lth, the processor 21 performs, for example, the output control in the second sealing mode (step S108).

In the present modification, when a branch of the blood vessel is detected in the observation image, the distance L between the branch of the blood vessel and the grasping position of the blood vessel is calculated, and whether or not the distance L is less than or equal to the predetermined distance Lth is judged. Consequently, whether or not the position of the detected branch is within the predetermined range from the grasping position of the blood vessel (the range which is at the predetermined distance Lth or less from the grasping position) is properly judged. Therefore, in the present modification as well, whether or not any branch of the blood vessel is present within the predetermined range from the grasping position of the blood vessel is properly judged.

Furthermore, the processing shown in each of FIGS. 3, 11, 14, and 15 has only to be performed in one of the processor 21 of the controller (energy controller) 3 and the processor 72 of the image processing device 65. For example, in a certain modification, the processor 21 of the controller 3 performs the detection processing of the branch of the blood vessel in the set detection range (step S104), and in another certain example, the processor 72 of the image processing device 65 judges whether or not any branch of the blood vessel is present within the predetermined range from the position where the blood vessel is grasped (the grasping position of the blood vessel) (step S106). In yet another certain modification, an integral device having both of the functions of the controller 3 and the image processing device 65 may be provided in the treatment system 1. In the present modification, the processing shown in each of FIGS. 3, 11, 14, and 15 is performed by a processor provided in this integral device.

In the embodiments and others described above, the energy treatment instrument (2) of the treatment system (1) comprises the first grasping piece (15), and the second grasping piece (16) which opens or closes relative to the first grasping piece (15) and which grasps a blood vessel between the first grasping piece (15) and the second grasping piece (16). Further, the actuation state of the energy treatment instrument (2) is switched between the first mode to coagulate the blood vessel when a branch is not present within the predetermined range from the position where the blood vessel is grasped, and the second mode to coagulate the blood vessel when a branch is present within the predetermined range. Further, in the treatment system (1), the energy output source (32; 47; 32, 47) outputs electric energy which is supplied to the energy treatment instrument (2), and the electric energy is supplied to the energy treatment instrument (2), whereby the treatment energy is applied to the blood vessel grasped between the first grasping piece (15) and the second grasping piece (16). The observation element (60) observes the grasped treated target. The processor (21, 72) judges, on the basis of the observation image in the observation element (60), whether or not any branch of the blood vessel is present within the predetermined range from the position where the blood vessel is grasped. The processor (21, 72) performs at least one of the following: controlling the output of the electric energy from the energy output source (32; 47; 32, 47) on the basis of a judgement result of the branch, and making the force of grasping the blood vessel between the first grasping piece (15) and the second grasping piece (16) greater when it is judged that a branch of the blood vessel is present than when it is judged that the branch of the blood vessel is not present.

Although the embodiments, etc. of the present invention have been described above, the present invention is not limited to the above-described embodiments, etc., and, needless to say, various modifications can be made without departing from the spirit of the invention.

Characteristic matters are additionally noted below.

### (Additional note 1)

A treatment method comprising:
closing a first grasping piece and a second grasping piece relative to each other, and grasping a blood vessel between the first grasping piece and the second grasping piece;
observing the grasped blood vessel;
supplying electric energy to an energy treatment instrument from an energy output source, and applying treatment energy to the treated target grasped between the first grasping piece and the second grasping piece;
judging, on the basis of the observation image of the blood vessel, whether or not any branch of the blood vessel is present within a predetermined range from a position where the blood vessel is grasped; and
performing at least one of the following: controlling the output of the electric energy from the energy output source on the basis of a judgement result of the branch, and making the force of grasping the blood vessel between the first grasping piece and the second grasping piece greater when it is judged that the branch is present than when it is judged that the branch is not present.

## Claims

1. An energy treatment instrument comprising:
a first grasping piece, and a second grasping piece which opens or closes relative to the first grasping piece and which grasps a blood vessel between the first grasping piece and the second grasping piece,
wherein an actuation state of the energy treatment instrument is switched between a first mode to coagulate the blood vessel when a branch of the blood vessel is not present within a predetermined range from a position where the blood vessel is grasped, and a second mode to coagulate the blood vessel when the branch of the blood vessel is present within the predetermined range.

2. The energy treatment instrument according to claim 1, wherein at least one of the first grasping piece and the second grasping piece includes an energy application section which applies treatment energy to the grasped blood vessel.

3. The energy treatment instrument according to claim 2, wherein the state of the application of the treatment energy to the grasped blood vessel from the energy application section varies between the first mode and the second mode.

4. The energy treatment instrument according to claim 1, wherein force of grasping the blood vessel between the first grasping piece and the second grasping piece varies between the first mode and the second mode.

5. A treatment system comprising:
the energy treatment instrument according to claim 1;
an observation element to observe the grasped blood vessel;
an energy output source which outputs electric energy that is supplied to the energy treatment instrument, and which applies treatment energy to the blood vessel grasped between the first grasping piece and the second grasping piece by the supply of the electric energy to the energy treatment instrument; and
a processor which judges, on the basis of an observation image in the observation element, whether or not any branch of the blood vessel is present within the predetermined range from the position where the blood vessel is grasped, and switches the actuation state of the energy treatment instrument between the first mode and the second mode by controlling the output of the electric energy from the energy output source on the basis of a judgement result of the branch.

6. The treatment system according to claim 5, wherein the processor makes the output electric energy lower and makes an output time of the electric energy longer when it is judged that the branch is present than when it is judged that the branch is not present.

7. The treatment system according to claim 5, wherein when it is judged that the branch is present, the processor causes the electric energy to be intermittently output more than one time by stopping the output of the electric energy after starting the output of the electric energy, and again starting the output of the electric energy after once stopping the output of the electric energy.

8. The treatment system according to claim 5, wherein the processor detects impedance between the first grasping piece and the second grasping piece, and when it is judged that the branch is not present, the processor stops the output of the electric energy on the basis of the fact that the impedance is equal to or more than a first impedance threshold, and when it is judged that the branch is present, the processor stops the output of the electric energy on the basis of the fact that the impedance is equal to or more than a second impedance threshold higher than the first impedance threshold.

9. The treatment system according to claim 5, wherein when it is judged that the branch is present, the processor continuously stops the output of the electric energy.

10. The treatment system according to claim 5, wherein the processor sets, as a detection range, a range which is at a predetermined distance or less from the position where the blood vessel is grasped in the observation image, and the processor detects the branch in the set detection range, and when the branch is detected in the detection range, the processor judges that the branch is present within the predetermined range from the position where the blood vessel is grasped.

11. The treatment system according to claim 5, wherein the processor detects the branch in the whole observation image, and when the branch is detected in the observation image, the processor calculates a distance between the detected branch and the position where the blood vessel is grasped, and when the calculated distance is less than or equal to a predetermined distance, the processor judges that the branch of the blood vessel is present within the predetermined range from the position where the blood vessel is grasped.

12. The treatment system according to claim 5, wherein the first grasping piece includes a first electrode,
the second grasping piece includes a second electrode, and
the energy output source passes a high-frequency current as the treatment energy through the blood vessel between the first grasping piece and the second grasping piece by supplying the output electric energy to the first electrode and the second electrode.

13. A treatment system comprising:
the energy treatment instrument according to claim 1;
an observation element to observe the grasped blood vessel; and
a processor which judges, on the basis of an observation image in the observation element, whether or not any branch of the blood vessel is present within the predetermined range from the position where the blood vessel is grasped, and switches the actuation state of the energy treatment instrument between the first mode and the second mode by making force of grasping the blood vessel between the first grasping piece and the second grasping piece greater when it is judged that the branch is present than when it is judged that the branch is not present.

14. A controller which is used together with an energy treatment instrument, the energy treatment instrument including a first grasping piece, and a second grasping piece which opens or closes relative to the first grasping piece and which grasps a blood vessel between the first grasping piece and the second grasping piece, the controller comprising:
an energy output source which outputs electric energy that is supplied to the energy treatment instrument, and which applies treatment energy to the blood vessel grasped between the first grasping piece and the second grasping piece by the supply of the electric energy to the energy treatment instrument; and
a processor which judges, on the basis of an observation image resulting from observation by an observation element, whether or not any branch of the blood vessel is present within the predetermined range from the position where the blood vessel is grasped, the processor performing at least one of the following: controlling the output of the electric energy from the energy output source on the basis of a judgement result of the branch, and making force of grasping the blood vessel between the first grasping piece and the second grasping piece greater when it is judged that the branch is present than when it is judged that the branch is not present.
